# EUROPÄISCHE PATENTSCHRIFT

(11) **EP 0 991 447 B1**
(45) Veröffentlichungstag und Bekanntmachung des Hinweises auf die Patenterteilung: **01.12.2004**
(21) Anmeldenummer: 98934909.7
(22) Anmeldetag: 03.06.1998
(51) Int. Cl.: A61N 7/00, A61H 23/00

(54) **MEDIZINISCHES INSTRUMENT ZUR BEHANDLUNG VON BIOLOGISCHEM GEWEBE**
MEDICAL INSTRUMENT FOR TREATING BIOLOGICAL TISSUE
INSTRUMENT MEDICAL POUR LE TRAITEMENT DE TISSUS BIOLOGIQUES

(30) Priorität: 17.06.1997 DE 19725477
(43) Veröffentlichungstag der Anmeldung: 12.04.2000
(73) Patentinhaber: Ferton Holding, 2800 Delemont (CH)
(72) Erfinder: HAUPT, Gerald, D-44577 Castrop (DE); MENNE, Andreas, D-88709 Meersburg (DE); SCHULZ, Manfred, D-88662 Überlingen (DE)
(74) Vertreter: Dallmeyer, Georg, Dipl.-Ing.
(86) Internationale Anmeldenummer: PCT/EP1998/003317
(87) Internationale Veröffentlichungsnummer: WO 1998/057707

(56) Entgegenhaltungen:
- EP-A- 0 317 507
- WO-A-92/09238
- WO-A-93/14720
- WO-A-93/16652
- FR-A- 1 062 415
- GB-A- 2 277 450
- US-A- 4 530 360
- US-A- 4 549 535
- US-A- 4 716 890

## Beschreibung

Die Erfindung betrifft ein medizinisches Instrument zur Behandlung von biologischem Gewebe.

Derartige Instrumente dienen dazu, mittels Druck- oder Stoßwellen den Heilungsprozeß bei Knochenbrüchen, Enthesiopathien, Tendopathien aber auch bei Parodontose zu beschleunigen. Ein weiteres Einsatzgebiet ist die Schmerztherapie im knochennahen Weichteilbereich des Haltungs- und Bewegungsapparates.

Bei bislang bekannten extrakorporalen Druckwellengeneratoren wird im Brennpunkt eines akustischen Reflektors, z.B. mittels einer Funkenentladung eine Druck- oder Stoßwelle erzeugt, die dann durch den Reflektor auf das zu beschallende Objekt fokussiert wird. Es wird vermutet, daß mit Hilfe der Druckwellen Mikroschädigungen im biologischen Gewebe erzeugt werden, die den Körper zu Regenerationsmaßnahmen veranlassen.

Bekannte Druckimpulsquellen verwenden fokussierte Stoßwellen und können nur im eng begrenzten Fokusbereich eine Wirkung erzielen. Für ein befriedigendes Behandlungsergebnis muß allerdings der gesamte Knochenbruchbereich gleichmäßig beschallt werden. Dies verlangt einen aufwendigen Bewegungsmechanismus für die Druckimpulsquelle und ist zudem durch das wiederholte Aufsuchen der Behandlungspositionen sehr zeitintensiv.

In dem Forschungsbericht T 83-285 des Bundesministeriums für Forschung und Technologie der Bundesrepublik Deutschland "Extrakorporale Stimulation des Herzens durch mechanische Reize - Entwicklung und Bau eines Gerätes zur klinischen Erprobung'', O. Wess et al, Dez. 1983 wird folgendes Instrument offenbart: Ein Instrument zur Behandlung von biologischem Gewebe mit einer Einrichtung zum extrakorporalen Erzeugen von Druckwellen und mit einem Übertragungselement zum Einkoppeln der Druckwellen in den Körper von Lebewesen, wobei das Übertragungselement eine stumpfe Sondenspitze mit einer flachen Austrittsgrenzfläche aufweist, die eine unfokussierte, mechanisch erzeugte Druckwellen in das biologische Gewebe einkoppelt, die von einem auf eine Endgeschwindigkeit von bis zu 2,5 m/s beschleunigten und auf das Übertragungselement auftreffenden hin und her bewegbaren Schlagteil erzeugt wird.

Auch aus der US-A-4, 549, 535 und der US-A-4,716,890 sind entsprechende Geräte bekannt, allerdings ohne Angabe der besagten Endgeschwindigkeit.

In der Schmerztherapie tritt noch ein weiteres Problem bei der Verwendung bekannter Druckimpulsquellen auf. Die während der Behandlung eingesetzten Ortungssysteme zur Lokalisierung des Behandlungsortes (Ultraschall und Röntgen) können die Schmerzquelle nicht konkret anzeigen und der behandelnde Arzt beschallt mit einer großen Anzahl von Einzelimpulsen den vermuteten Schmerzherd.

Der Erfindung liegt demzufolge die Aufgabe zugrunde, einen Druckwellengenerator so auszubilden, daß er auf eine einfache und kostengünstige Weise eine gleichmäßige Energieverteilung der Druckwellen auf einen großflächigen Wirkungsbereich ermöglicht.

Zur Lösung dieser Aufgabe dienen die Merkmale des Anspruchs 1.

Die Erfindung sieht in vorteilhafter Weise vor, daß das Übertragungselement eine stumpfe Sondenspitze mit einer flachen oder konkav gekrümmten Austrittsfläche aufweist, die eine unfokussierte, mechanisch erzeugte Druckwelle in das biologische Gewebe durch Längenänderung des Übertragungselementes einkoppelt, die von einem auf eine hohe Endgeschwindigkeit von über 5 m/s beschleunigten und auf das Übertragungselement auftretenden Schlagteil (10) erzeugt wird. Die Druckwelle kann sich bis zu ihrem Applikationsort ausbreiten, ohne daß das Übertragungselement in direktem Kontakt mit dem Applikationsort steht. Die Erfindung zielt darauf ab, die Druckwellen nicht zu fokussieren und es dadurch zu ermöglichen, daß diese großflächig eingekoppelt werden können. Das medizinische Instrument eignet sich insbesondere für Behandlungen, bei denen die Sondenspitze auf der Körperoberfläche sehr nah an dem Applikationsort angeordnet werden kann, wie dies beispielsweise bei einem Tennisellenbogen, einem Fersensporn oder auch bei Parodontose der Fall ist.

Vorzugsweise ist vorgesehen, daß die Einrichtung zum Erzeugen der Druckwelle aus einem in einem Gehäuse geführten, mit Hilfe eines Antriebsmittels hin- und herbewegbaren Schlagteil besteht, das auf das Übertragungselement einen oder mehrere Kraftstöße ausübt, wobei das Schlagteil infolge des Kraftstoßes eine Druckwelle in das Übertragungselement induziert, die sich bis zu der Austrittsgrenzfläche der stumpfen Sondenspitze des Übertragungselementes fortpflanzt. Die Druckwelle wird demzufolge in einfacher Weise mechanisch erzeugt. Durch die schnelle Bewegung der Sondenspitze läßt sich eine Druckwelle mit hohen Druckspitzenwerten erzeugen. Die Druckwelle pflanzt sich in dem biologischen Gewebe fort und wird nicht fokussiert. Die mit einem solchen System erzeugten Druckwellen erreichen nicht die kurzen Anstiegszeiten der Druckwellengeneratoren mit fokussierter Druckwelle, sind aber in der maximalen Druckspitze nicht wesentlich schwächer. Die unfokussierte Druckwelle breitet sich bis zum Anwendungsort in dem biologischen Gewebe radial aus.

Wesentliche Vorteile der Erfindung bestehen darin, daß das medizinische Instrument einen einfachen, kostengünstigen Aufbau aufweist, dessen Kosten lediglich einen Bruchteil bisher bekannter Druckwellengeneratoren beträgt.

Das medizinische Instrument kann als kleines transportables Gerät ausgebildet sein, das leichter applizierbar ist und ohne Behinderungen auf die zu behandelnde Körperstelle aufgesetzt werden kann. Das Gerät benötigt keine Verbrauchsmaterialien und insbesondere keine Ortungsvorrichtungen, da der Behandlungsbereich in der Nähe der Sondenspitze liegt.

Das Schlagteil ist vorzugsweise koaxial zu dem Übertragungselement angeordnet. Auf diese Weise kommt es zu einem direkten Impulsaustausch zwischen dem Schlagteil und dem Übertragungselement beim Aufschlagen des Schlagteils auf die Eintrittsgrenzfläche des Übertragungselementes.

Insbesondere bei orthopädischen Anwendungen ist es vorteilhaft, eine Vielzahl von einzelnen Druckwellen in das biologische Gewebe einzukoppeln, um eine optimale Wirkung zu erzielen. Das Antriebsmittel ist daher vorzugsweise so ausgestaltet, daß eine periodische Hin- und Herbewegung des Schlagteils möglich ist. Die Schlagfrequenz beträgt ca. 1 bis 30 Hz, vorzugsweise ca. 6 bis 20 Hz.

In einer bevorzugten Ausführungsform ist das Übertragungselement axial und linear in dem Gehäuse geführt, wobei eine in Axialrichtung wirkendes Feder-Dämpfungselement zwischen dem Übertragungselement und dem Gehäuse angeordnet ist. Auf diese Weise wird eine Entkopplung des Übertragungselementes von dem Gehäuse in Axialrichtung realisiert. Außerdem bringt dieses Feder-Dämpfungselement das Übertragungselement wieder in seine Ausgangslage nach jeder Druckwelle zurück und begrenzt dessen Auslenkung. Für die Einkopplung der Druckwelle in das biologische Gewebe ist eine große Auslenkung der Ausgangsgrenzfläche des Übertragungselementes nicht notwendig. Die Einkopplung der Druckwelle soll sogar nach Möglichkeit nur aufgrund der Längenänderung des Übertragungselementes und nicht durch dessen Verlagerung erfolgen.

Die Sondenspitze kann aufgrund der Kraftstöße auf die Eintrittsgrenzfläche des Übertragungselementes einen Hub von weniger als 1 mm, vorzugsweise weniger als 0,5 mm, ausführen.

Zwischen dem Schlagteil und dem Übertragungselement kann ein Zwischenelement angeordnet sein, das den Kraftstoß von dem Schlagteil auf das Übertragungselement weiterleitet. Dieses Zwischenelement kann dazu dienen, eine bessere Abschirmung der Antriebsmittel gegenüber dem Applikationsbereich zu schaffen oder zum Umlenken der Richtung der Druckwelle oder auch zum Beeinflussen der Druckwellencharakteristik dienen.

An dem proximalen Ende der Führung des Schlagteils kann ein Magnethalter für das Schlagteil angeordnet sein, der das Schlagteil in der proximalen Endlage hält, bis es von den Antriebsmitteln erneut beschleunigt wird.

Die stumpfe Sondenspitze weist vorzugweise eine flache Austrittsgrenzfläche mit abgerundeten Kanten auf. Die Austrittsgrenzfläche des Übertragungselementes ist möglichst großflächig, um einen hohen Wirkungsgrad bei der Übertragung der Druckwelle zu erzielen. Die gerundeten Kanten vermeiden Verletzungen der Hautoberfläche.

Die Sondenspitze kann auch eine Austrittsgrenzfläche aufweisen, die konkav gekrümmt ist und ebenfalls mit gerundeten Kanten versehen ist.

Bei einem weiteren Ausführungsbeispiel des Übertragungselementes kann vorgesehen sein, daß die Austrittsgrenzfläche einen erheblich größeren Durchmesser aufweist als die Eintrittsgrenzfläche. Eine derartige Austrittsgrenzfläche gewährleistet eine große Übertragungsfläche für die Einkopplung der Druckwelle, so daß die eingekoppelte spezifische Druckwellenenergie zur Schonung der Hautoberfläche reduziert ist.

Zwischen der Sondenspitze und der Einkoppelstelle auf dem biologischen Gewebe kann ein Impedanzanpassungsmedium angeordnet sein, das die Einkopplung der Druckwelle in das biologische Gewebe verbessert. Ein geeignetes pastenförmiges Impedanzanpassungsmedium ist beispielsweise ein Ultraschallgel oder eine andere pastöse Masse, z.B. Vaseline.

Das Übertragungselement kann zur Impedanzanpassung aus unterschiedlichen Materialien aufgebaut sein, die das Übertragungsverhalten verbessern. Durch eine geeignete Materialauswahl kann das Übertragungsverhalten der Druckwelle bzw. des Übertragungselementes und damit die Einkopplung in das biologische Gewebe beeinflußt werden. Das einstückige Übertragungselement aus unterschiedlichen Materialien aufzubauen, hat das Ziel, die Druckwelle möglichst verlustarm in den Körper einzukoppeln, und dadurch eine Impedanzanpassung zu ermöglichen.

Die Länge des Übertragungselementes kann im Bereich zwischen ca. 20 und 100 mm liegen. Über unterschiedliche und auswechselbare Übertragungselemente kann eine Anpassung an die gewünschte Behandlung erfolgen.

Im folgenden werden unter Bezugnahme auf die Zeichnungen Ausführungsbeispiele der Erfindung näher erläutert.

Es zeigen:
- Fig. 1: eine Darstellung des medizinischen Instrumentes im Querschnitt, und
- Fign.2: alternative Ausführungsformen des Übertragungs- und3elementes.

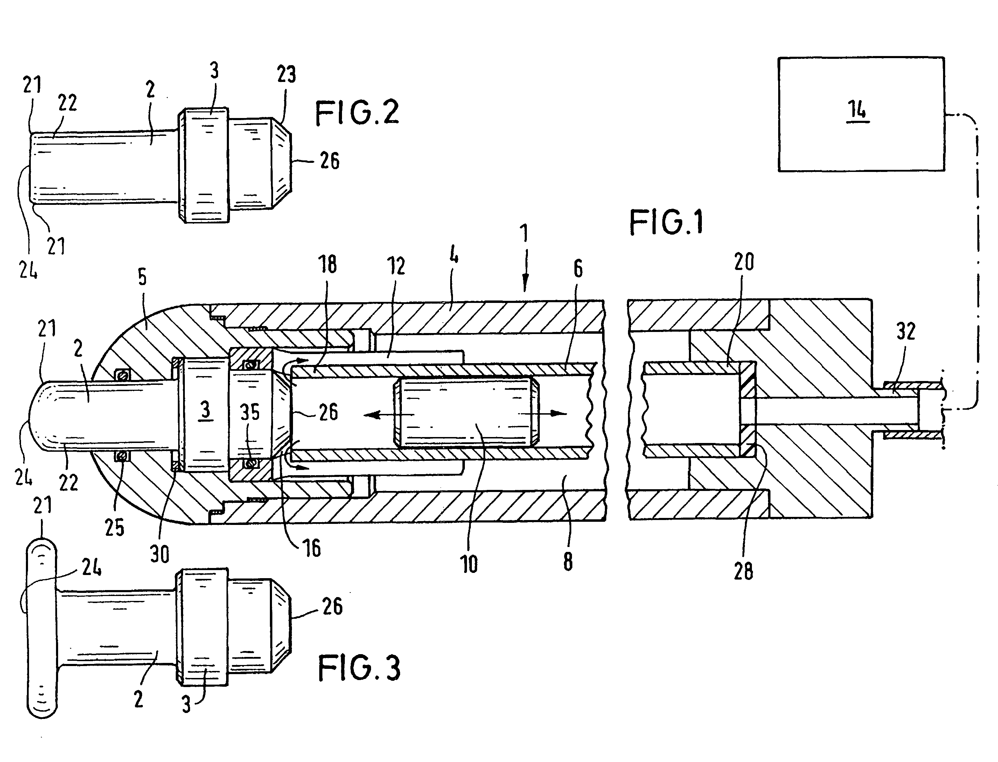

Das in Fig. 1 gezeigte Handstück 1 besteht aus einem Gehäuse 4, das einen pneumatischen Zylinder 6 aufnimmt, in dem ein Schlagteil 10 mit Hilfe pneumatischer Antriebsmittel 14 in Verbindung mit einer Staudruckkammer 8, die den Zylinder 6 koaxial ringförmig umgibt, zwischen zwei Endpositionen hin- und herbewegt wird. Alternativ ist es auch möglich, das Schlagteil 10 hydraulisch, mechanisch, elektromagnetisch oder durch andere Antriebsmittel zu beschleunigen. Bei elektromagnetischer Beschleunigung des Schlagteils 10 kann der Beschleunigungsweg verkürzt werden, der bei einem pneumatischen Antrieb ca. 100 bis 200 mm Länge aufweist.

In der proximalen Endposition des Schlagteils 10 ist am proximalen Ende 20 des Zylinders 6 ein Magnethalter 28 angeordnet, der das metallische Schlagteil 10 in seiner proximalen Endposition festhalten kann bis erneut ein über den Anschluß 32 aufgebrachter pneumatischer Druck das Schlagteil 10 in Richtung auf das distale Ende 18 des Zylinders 6 beschleunigt. Die sich in Bewegungsrichtung des Schlagteils 10 vor dem Schlagteil befindliche Luft wird über einen an dem distalen Ende 18 des Zylinders 6 befindlichen Ringschlitz 16 in die Staudruckkammer 8 geleitet. Durch die Beschleunigung des Schlagteils 10 trifft dieses mit einer hohen Endgeschwindigkeit von 5 bis 20 m/s auf eine distal von dem Zylinder 6 angeordnete Eintrittsgrenzfläche 26 eines Übertragungselementes 2. Das Übertragungselement 2 besteht aus einer metallischen Sonde, mit einer stumpfen Sondenspitze 22 und einer planen oder konvex geformten Austrittsgrenzfläche 24. Das Schlagteil 10 übt einen oder mehrere Kraftstöße auf das Übertragungselement 2 aus, das die von dem Schlagteil 10 induzierte Druckwelle an die Austrittsgrenzfläche 24 weiterleitet und dort in ein biologisches Gewebe einkoppelt.

Das Übertragungselement 2 ist in dem Gehäuse 4 linear und vorzugsweise koaxial zu dem Schlagteil 10 geführt. Das Gehäuse 4 weist ein Kopfteil 5 auf, das für ein Auswechseln des Übertragungselementes 2 abschraubbar ist. Das Übertragungselement 2 ist in einer Bohrung des Kopfteils 5 gelagert und mit Hilfe eines O-Rings 25 im vorderen Abschnitt des Kopfteils 5 abgedichtet. Ein Ringbund 3 des Übertragungselementes 2 dient als Anschlagelement wobei zwischen dem Ringbund 3 des Übertragungselementes 2 und dem Kopfteil 5 des Gehäuses 4 ein Feder/Dämpfungselement 30 angeordnet ist, das das Übertragungselement 2 von dem Gehäuse 4 in Axialrichtung entkoppelt. Das Feder/Dämpfungselement 30 hat aber auch eine Rückstellfunktion für das Übertragungselement 2 und drückt dieses nach jeder Druckwelle in seine proximale Ausgangslage zurück. Zugleich begrenzt es dessen Verlagerung während einer Druckwelle. Für die Einkopplung der Druckwelle in das biologische Gewebe ist eine Verlagerung des Übertragungselementes nicht notwendig und sogar unerwünscht, um Verletzungen zu vermeiden. Die Druckwelle soll allein durch die Längenänderung des Übertragungselementes 2 infolge der Druckwelle in das biologische Gewebe eingekoppelt werden.

Der sich in der Staudruckkammer 80 aufbauende Staudruck genügt bei Wegfall des an dem pneumatischen Anschluß 32 anliegenden Druckes, um das Schlagteil 10 von der distalen Endlage an dem Übertragungselement 2 in die proximale Endlage zu dem Magnethalter 28 zurückzubewegen. Der pneumatische Druck an dem Anschluß 32 kann bis zu 5 bar betragen. Das Schlagteil 10 kann zwecks Anpassung an bestimmte Längen des Übertragungselementes 2 oder zum Erzeugen einer bestimmten Charakteristik der Druckwelle hinsichtlich Länge, Masse und maximale Aufschlaggeschwindigkeit unterschiedlich gewählt werden.

Die proximale Eintrittsgrenzfläche 26 des Übertragungselementes 2 weist in etwa den gleichen Druchmesser wie das Schlagteil 10 auf. Dagegen kann die Austrittsgrenzfläche 24 einen beispielsweise um den Faktor 2 größeren Durchmesser aufweisen als die Eintrittsgrenzfläche 26. Die Länge des Schlagteils 10 ist vorzugsweise größer als sein Durchmesser. Damit werden bessere Führungseigenschaften in dem Zylinder 6 erreicht. Außerdem kann mit Hilfe einer unterschiedlichen Länge des Schlagteils 10 die Masse in einfacher Weise variiert werden, ohne daß der Durchmesser des Zylinders 6 und der Eintrittsgrenzfläche 26 des Übertragungselementes 2 verändert werden müssen.

Das proximale Ende des Übertragungselementes 2 ist in einer geschlitzten Hülse 12 geführt und in dieser mit Hilfe eines O-Rings 35 radial abgedichtet. Die Hülse 12 bildet mit dem proximalen konischen Ende des Übertragungselementes 2 eine Verbindung zwischen der Staudruckkammer 8 und dem distal von dem Schlagteil 10 liegenden Hohlraum des Zylinders 6.

Die Druckwellen werden mit einer Schlagfrequenz von ca. 1 bis 30 Hz, vorzugsweise 6 bis 20 Hz, erzeugt. Die Sondenspitze 22 führt einen Bewegungshub aus, der maximal ca. 1 mm und vorzugsweise weniger als 0,5 mm beträgt.

Fig. 2 zeigt ein Übertragungselement 2 mit einer flachen, stumpfen Sondenspitze 22 mit abgerundeten Kanten.

Bei dem Ausführungsbeispiel des Übertragungselementes 2 gemäß Fig. 3 ist die Austrittsgrenzfläche 24 im Vergleich zur Eintrittsgrenzfläche 26 erheblich vergrößert. Das Durchmesserverhältnis der Austrittsgrenzfläche 24 zu der Eintrittsgrenzfläche 26 beträgt dabei ca. 2 bis 3.

Das medizinische Instrument ermöglicht es, ein therapeutisches Verfahren zum Behandeln von biologischem Hart- und Weichgewebe, insbesondere zur Heilung von Knochenleiden, wie Knochenbrüchen, Enthesiopathien, Tendopathien und Parodontose, sowie eine Schmerztherapie im knochennahen Weichteilbereich des Haltungs- und Bewegungsapparates, durchzuführen, indem mechanisch erzeugte Druckwellen unfokussiert über eine stumpfe Sonde mit flacher Austrittsgrenzfläche in das biologische Gewebe eingekoppelt werden.

Das Übertragungselement 2 kann aus unterschiedlichen Materialien bestehen, um die Druckwelle möglichst verlustarm in das biologische Gewebe einzukoppeln und dadurch eine Impedanzanpassung zu erzielen. Dabei kann vorgesehen sein, daß die unterschiedlichen Materialien in Axialrichtung hintereinander angeordnet sind.

## Patentansprüche

1. Medizinisches Instrument zur Behandlung von biologischem Gewebe, mit einer Einrichtung zum extrakorporalen Erzeugen von Druckwellen und mit einem Übertragungselement (2) zum Einkoppeln der Druckwellen in den Körper von Lebewesen, wobei
das Übertragungselement (2) eine stumpfe Sondenspitze (22) mit einer flachen oder gekrümmten Austrittsgrenzfläche (24) aufweist, die eine unfokussierte, mechanisch erzeugte Druckwelle in das biologische Gewebe einkoppelt, die von einem auf eine Endgeschwindigkeit von 5 m/s bis 20 m/s beschleunigten und auf das Übertragungselement (2) auftreffenden hin und her bewegbaren Schlagteil (10) erzeugt wird.

2. Medizinisches Instrument nach Anspruch 1, **dadurch gekennzeichnet, daß** das Übertragungselement (2) aus einer metallischen Sonde besteht.

3. Medizinisches Instrument nach einem der Ansprüche 1 oder 2, **dadurch gekennzeichnet, daß** die Sondenspitze (22) aufgrund der von dem Schlagteil (10) ausgeübten Kraftstöße einen Hub von weniger als 1 mm, vorzugsweise von weniger als 0,5 mm ausführt.

4. Medizinisches Instrument nach einem der Ansprüche 1 bis 3, dadurch gekennnzeichnet, daß die Einrichtung zum Erzeugen von Druckwellen aus einem in einem Gehäuse (4) geführten mit Hilfe eines Antriebsmittels (14) hin- und her bewegbaren Schlagteil (10) besteht, das auf das Übertragungselement (2) einen oder mehrere Kraftstöße ausübt, wobei das Schlagteil (10) infolge des Kraftstoßes eine Druckwelle in das Übertragungselement (2) induziert, die sich bis zu der Austrittgrenzfläche (24) der stumpfen Sondenspitze (22) des Übertragungselementes (2) fortpflanzt.

5. Medizinisches Instrument nach einem der Ansprüche 1 bis 4, **dadurch gekennzeichnet, daß** das Schlagteil (10) koaxial zu dem Übertragungselement (2) angeordnet ist.

6. Medizinisches Instrument nach Anspruch 1 bis 5, **dadurch gekennzeichnet, daß** die Schlagfrequenz des Schlagteils (10) ca. 1 bis 30 Hz, vorzugsweise 6 bis 20 Hz, beträgt.

7. Medizinisches Instrument nach Anspruch 1 oder 2, **dadurch gekennzeichnet, daß** das Schlagteil (10) für ein wiederholtes Anschlagen an das Übertragungselement (2) periodisch hin- und herbewegbar ist, wobei das Schlagteil (10) und das Übertragungselement selbsttätig rückstellbar sind.

8. Medizinisches Instrument nach einem der Ansprüche 1 bis 5, **dadurch gekennzeichnet, daß** zwischen Übertragungselement (2) und dem Gehäuse (4) ein in Axialrichtung wirkendes Feder/Dämpfungselement (30) angeordnet ist.

9. Medizinisches Instrument nach einem der Ansprüche 1 bis 8, **dadurch gekennzeichnet, daß** zwischen Schlagteil (10) und dem Übertragungselement (2) ein Zwischenelement angeordnet ist, das den Kraftstoß von dem Schlagteil (10) auf das Übertragungselement (2) weiterleitet.

10. Medizinisches Instrument nach einem der Ansprüche 1 bis 9, **dadurch gekennzeichnet, daß** an dem proximalen Ende (20) der Führung (6) des Schlagteils ein Magnethalter (28) für das Schlagteil (10) angeordnet ist.

11. Medizinisches Instrument nach einem der Ansprüche 1 bis 10, **dadurch gekennzeichnet, dass** das Übertragungselement (2) an der Austrittsgrenzfläche (24) einen größeren Durchmesser aufweist als an der Eintrittsgrenzfläche (26).

12. Medizinisches Instrument nach Anspruch 11, **dadurch gekennzeichnet, dass** das Durchmesserverhältnis der Austrittsgrenzfläche (24) zur der Eintrittsgrenzfläche (26) ca. 2 bis 3 beträgt.

13. Medizinisches Instrument nach einem der Ansprüche 1 bis 12, **dadurch gekennzeichnet, dass** zwischen der Sondenspitze (22) und der Einkoppelstelle auf dem biologischen Gewebe ein beispielsweise pastenförmiges Impedanzanpassungsmedium angeordnet ist, das die Einkopplung der Druckwelle in das biologische Gewebe verbessert.

14. Medizinisches Instrument nach einem der Ansprüche 1 bis 13, **dadurch gekennzeichnet, dass** das Übertragungselemente (2) in einem Gehäuse (4) linear geführt ist.

15. Medizinisches Instrument nach Anspruch 14, **dadurch gekennzeichnet, dass** das Gehäuse (4) zum Auswechseln des Übertragungselementes (2) einen abschraubbaren Kopfteil (5) aufweist.

## Claims

1. Medical instrument for treating biological tissue, comprising a device for generating extracorporeal pressure waves and a transmission element (2) for coupling pressure waves into the bodies of living organisms, wherein
the transmission element (2) has a blunt probe tip (22) with a flat or curved exit surface (24), which couples a non-focused mechanically generated pressure wave into the biological tissue, said pressure wave being generated by a reciprocable beater portion (10) accelerated to a final velocity of more than 5 m/s to 20 m/s and striking onto the transmission element (2).

2. Medical instrument according to claim 1, **characterized in that** the transmission element (2) comprises a metal probe.

3. Medical instrument according to one of claims 1 or 2, **characterized in that** the probe tip (22) performs, due to the impulses from the beater portion (10), a stroke of less that 1 mm, preferably less than 0.5 mm.

4. Medical instrument according to one of claims 1 to 3, **characterized in that** the device for generating pressure waves comprises a beater portion (10) capable of being reciprocated with the aid of an actuating means (14) and guided in a housing (4), said beater portion (10) exerting one, or a plurality of impulses on the transmission element (2), wherein the beater portion (10), as a result of the impulse, induces a pressure wave into the transmission element (2), said pressure wave propagating up to the exit interface (24) of the blunt probe tip (22) of the transmission element (2).

5. Medical instrument according to one of claims 1 to 4, **characterized in that** the beater portion (10) is arranged coaxially with the transmission element (2).

6. Medical instrument according to claim 1 to 5, **characterized in that** the beating frequency of the beater portion (10) amounts to approximately 1 to 30 Hz, preferably to 6 to 20 Hz.

7. Medical instrument according to claim 1 or 2, **characterized in that** the beater portion (10) is capable of being periodically reciprocated for the purpose of repeatedly striking onto the transmission element (2), wherein the beater portion (10) and the transmission element are automatically resettable.

8. Medical instrument according to one of claims 1 to 5, **characterized in that** a spring/damping element (30) acting in axial direction is arranged between the transmission element (2) and the housing (4).

9. Medical instrument according to one of claims 1 to 8, **characterized in that** between the beater portion (10) and the transmission element (2) an intermediate element is arranged which transmits the impulse from the beater portion (10) to the transmission element (2).

10. Medical instrument according to one of claims 1 to 9, **characterized in that** a magnetic holder (28) for the beater portion (10) is arranged at the proximal end (20) of the guide (6) of the beater portion.

11. Medical instrument according to one of claims 1 to 10, **characterized in that** the transmission element (2) has a larger diameter at the exit interface (24) than at the entry interface (26).

12. Medical instrument according to claim 11, **characterized in that** the diameter ratio of the exit interface (24) to the entry interface (26) amounts to approximately 2 to 3.

13. Medical instrument according to one of claims 1 to 12, **characterized in that** between the probe tip (22) and the coupling location on the biological tissue an e. g. pasty impedance-matching medium is located which improves coupling of the pressure wave into the biological tissue.

14. Medical instrument according to one of claims 1 to 13, **characterized in that** the transmission element (2) is linearly guided in a housing (4).

15. Medical instrument according to claim 14, **characterized in that** the housing (4) comprises, for the purpose of exchanging the transmission element (2), a screw-off type head portion (5).

## Revendications

1. Instrument médical destiné au traitement de tissus biologiques, lequel instrument comporte un dispositif, destiné à générer à l'extérieur du corps des ondes de pression, et un élément de transmission (2) destiné à injecter les ondes de pression dans le corps d'êtres vivants,
l'élément de transmission (2) comportant une pointe de sonde (22) épointée présentant une surface limite de sortie (24) plate ou incurvée qui injecte une onde de pression non focalisée, générée mécaniquement, dans le tissu biologique, laquelle onde de pression est générée par une pièce de percussion (10) mobile suivant un mouvement alternatif incident l'élément de transmission (2) et accélérée à une vitesse finale comprise entre 5 m/s et 20 m/s.

2. Instrument médical selon la revendication 1, **caractérisé en ce que** l'élément de transmission (2) est constitué par une sonde métallique.

3. Instrument médical selon l'une des revendications 1 ou 2, **caractérisé en ce que** la pointe de sonde (22) effectue, en raison d'impulsions générées par la pièce de percussion (10), une course de moins de 1 mm, avantageusement de moins de 0,5 mm.

4. Instrument médical selon l'une des revendications 1 à 3, **caractérisé en ce que** le dispositif destiné à générer des ondes de pression est constitué d'une pièce de percussion (10) qui est guidée dans un boîtier (4), qui est mobile suivant un mouvement alternatif à l'aide d'un moyen d'entraînement (14) et qui génère une ou plusieurs impulsions, la pièce de percussion (10) induisant à la suite de l'impulsion dans l'élément de transmission (2) une onde de pression qui se propage jusqu'à la surface limite de sortie (24) de la pointe de sonde épointée (22) de l'élément de transmission (2).

5. Instrument médical selon l'une des revendications 1 à 4, **caractérisé en ce que** la pièce de percussion (10) est placée coaxialement à l'élément de transmission (2).

6. Instrument médical selon la revendication 1 à 5, **caractérisé en ce que** la fréquence de percussion de la pièce de percussion (10) est comprise entre 1 et 30 Hz environ, avantageusement entre 6 et 20 Hz.

7. Instrument médical selon la revendication 1 ou 2, **caractérisé en ce que** la pièce de percussion (10) est mobile suivant un mouvement alternatif périodique afin de générer des percussions répétées contre l'élément de transmission (2), la pièce de percussion (10) et l'élément de transmission étant à rappel automatique.

8. Instrument médical selon l'une des revendications 1 à 5, **caractérisé en ce qu'**un élément d'amortissement ou à ressort (30), agissant axialement, est placé entre l'élément de transmission (2) et le boîtier (4).

9. Instrument médical selon l'une des revendications 1 à 8, **caractérisé en ce qu'**un élément intermédiaire est placé entre la pièce de percussion (10) et l'élément de transmission (2) et transmet l'impulsion de la pièce de percussion (10) à l'élément de transmission (2).

10. Instrument médical selon l'une des revendications 1 à 9, **caractérisé en ce qu'**un support magnétique (28) destiné à la pièce de percussion (10) est placé à l'extrémité proximale (20) du guide (6) de la pièce de percussion.

11. Instrument médical selon l'une des revendications 1 à 10, **caractérisé en ce que** l'élément de transmission (2) présente au niveau de la surface limite de sortie (24) un diamètre qui est supérieur au diamètre au niveau de la surface limite d'entrée (26).

12. Instrument selon la revendication 11, **caractérisé en ce que** le rapport entre le diamètre au niveau la surface limite de sortie (24) et le diamètre au niveau de la surface limite d'entrée (26) est compris entre 2 et 3 environ.

13. Instrument médical selon l'une des revendications 1 à 12, **caractérisé en ce qu'**un médium d'adaptation d'impédance, qui se présente par exemple sous la forme d'une pâte et qui améliore l'injection des ondes de pression dans le tissu biologique, est placé entre la pointe de sonde (22) et l'emplacement d'injection dans le tissu biologique.

14. Instrument médical selon l'une des revendications 1 à 13, **caractérisé en ce que** l'élément de transmission (2) est guidé linéairement dans un boîtier (4).

15. Instrument médical selon la revendication 14, **caractérisé en ce que** le boîtier (4) comporte une pièce de tête (5) dévissable afin de remplacer l'élément de transmission (2).
